**Europäisches Patentamt**

**European Patent Office**

**Office européen des brevets**

(19)

(11) Veröffentlichungsnummer : **0 318 504 B1**

# EUROPÄISCHE PATENTSCHRIFT

(12)

(45) Veröffentlichungstag der Patentschrift :
**12.06.91 Patentblatt 91/24**

(51) Int. Cl.$^5$ : **C07C 7/04, F25J 3/02**

(21) Anmeldenummer : **87905437.7**

(22) Anmeldetag : **04.08.87**

(86) Internationale Anmeldenummer :
**PCT/EP87/00427**

(87) Internationale Veröffentlichungsnummer :
**WO 88/00936 11.02.88 Gazette 88/04**

(54) **Verfahren zum Abtrennen höherer Kohlenwasserstoffe aus einem Gasgemisch.**

(30) Priorität : **06.08.86 DE 3626560**
**06.08.86 DE 3626561**

(43) Veröffentlichungstag der Anmeldung :
**07.06.89 Patentblatt 89/23**

(45) Bekanntmachung des Hinweises auf die
Patenterteilung :
**12.06.91 Patentblatt 91/24**

(84) Benannte Vertragsstaaten :
**DE FR GB NL SE**

(56) Entgegenhaltungen :
**GB-A- 2 110 808**

(73) Patentinhaber : **Linde Aktiengesellschaft**
**Abraham-Lincoln-Strasse 21**
**W-6200 Wiesbaden (DE)**

(72) Erfinder : **BAUER, Heinz**
**Nibelungenstrasse 6**
**W-8000 München 19 (DE)**

(74) Vertreter : **Schaefer, Gerhard, Dr.**
**Linde Aktiengesellschaft Zentrale**
**Patentabteilung**
**W-8023 Höllriegelskreuth (DE)**

## Beschreibung

Die Erfindung betrifft ein Verfahren zum Abtrennen höherer Kohlenwasserstoffe aus einem diese und leichter siedende Komponenten enthaltenden Gasgemisch durch rektifikatorische Zerlegung, indem das Gasgemisch partiell kondensiert und einer Trennsäule zugeleitet wird, an deren Sumpf eine an höheren Kohlenwasserstoffen reiche Fraktion und an deren Kopf eine an leichter siedenden Komponenten reiche Fraktion abgezogen werden, wobei ein Teil der Kopffraktion kondensiert und als Rücklauf auf den Kopf der Trennsäule gegeben wird.

Die partielle Kondensation stellt einen einfachen Trennprozeß dar, bei dem ein zu zerlegender Gasstrom lediglich durch Temperaturabsenkung bis unter den Taupunkt und anschließender Phasentrennung in einem Abscheider in zwei Fraktionen unterschiedlicher Zusammensetzung zerlegt wird. Es handelt sich dabei um eine unscharfe Trennung mit lediglich einer Gleichgewichtsstufe, bei der im Kondensat höhersiedende und in der Gasphase leichtersiedende Komponenten angereichert sind. Wegen der geringen Trennschärfe kommen derartige Verfahren im wesentlichen als Vortrennstufen vor einer größeren Fraktioniereinheit zum Einsatz, beispielsweise vor einer Trennsäule.

Eine schärfere Trennung, die allerdings mit erhöhtem Aufwand erkauft werden muß, ist beispielsweise bei Einsatz eines Dephlegmators bzw. Rückflußkühlers möglich. In einem Rückflußkühler wird das aufwärtsströmende Gasgemisch durch indirekten Wärmetausch mit einem Kühlmedium, vorzugsweise dem kalten, nicht kondensierten Gas, gekühlt, wobei mit zunehmender Abkühlung immer mehr Bestandteile des Gasgemisches kondensieren und in Gegenrichtung zum Gasstrom nach unten absinken. Die herabfallende Flüssigkeit tritt dabei in Wärme- und Stoffaustausch mit dem aufsteigenden Gasgemisch, so daß sich innerhalb des Rückflußkühlers eine rektifikatorische Gastrennung abspielt. Die abzutrennende schwere Komponente kann vom Sumpf des Rückflußkühlers abgezogen werden, während das weitgehend von der schweren Komponente befreite, oben aus dem Rückflußkühler austretende kalte Gas beispielsweise nach einer kälteleistenden Entspannung im Gegenstrom zum zu zerlegenden Gasgemisch als Kühlmittel durch den Rückflußkühler zugeführt werden kann, wo es durch indirekten Wärmetausch näherungsweise wieder auf die Ausgangstemperatur angewärmt wird, bevor es einer anderen Verwendung zugeführt wird. Im Rückflußkühler sind üblicherweise für die Abkühlung des Gasgemisches Wärmetauscher eingebaut, die aufgrund ihrer speziellen Ausführung, beispielsweise als gewickelte Wärmetauscher oder als Plattenwärmetauscher, einen intensiven Kontakt zwischen dem aufwärtsströmenden Gasgemisch und der herabfallenden Flüssigkeit bewirken.

Aus der EP-A 126 309 ist z.B. ein derartiges Verfahren bekannt, bei den ein höchstens 10% $C_4$-Kohlenwasserstoffe und mindestens 65% Methan und leichtere Komponenten enthaltendes Gasgemisch unter Verwendung eines Dephlegmators oder Rückflußkühlers zerlegt wird.

Die Verwendung eines Rückflußkühlers anstelle einer üblichen Rektifiziersäule bietet insbesondere unter thermodynamischen Gesichtspunkten Vorteile, da der für die Rektifikation des Gasgemisches benötigte Rücklauf innerhalb des Rückflußkühlers selbst bei gleitender Temperatur erzeugt wird, während bei einer Rektifiziersäule der gesamte Rücklauf bei tiefster Temperatur bereitgestellt und auf den Säulenkopf aufgegeben werden muß. Die Tatsache, daß der Rücklauf bei gleitender Temperatur aus dem Gasgemisch selbst gebildet wird, kann in speziellen Fällen aber auch gegen die Verwendung eines Rücklaufkondensators sprechen. Wenn beispielsweise das zu zerlegende Gasgemisch im wesentlichen aus Komponenten mit weit auseinanderliegenden Siedepunkten besteht, kondensiert kurz unter dem Taupunkt im unteren Bereich des Rückflußkühlers bereits ein großer Teil der höhersiedenden Komponente. Soll diese Komponente jedoch in hoher Ausbeute abgetrennt werden, müssen Temperaturen weit unter dem Taupunkt eingestellt werden, beispielsweise bis zu 100°C unter dem Taupunkt. Ein solcher ungleichmäßiger Kondensationsverlauf ist nicht günstig, weil bei sinkender Temperatur immer weniger Kondensat neu gebildet, also immer weniger Rücklauf für den oberen Bereich des Rückflußkühlers erzeugt wird. Dies hat zur Folge, daß die Rektifikation ineffektiv und die gesamte Rücklaufkondensation in Frage gestellt wird.

Aus der britischen Patentanmeldung GB 2,110,808 ist ein Verfahren zur Abtrennung von $C_{2+}$-Komponenten aus einem Erdgasstrom bekannt. Dazu wird der Erdgasstrom partiell kondensiert und einer Trennsäule zugeleitet. An deren Sumpf wird eine $C_{2+}$-Fraktion abgezogen, die in einem Demethanizer von darin enthaltenem Methan befreit wird. Die Kopffraktion des Demethanizers wird zur Entfernung noch vorhandener $C_{2+}$-Komponenten in die Trennsäule zurückgeleitet. Vom Kopf der Trennsäule wird ein methanreiches Gas abgezogen, von dem ein Teil abgezweigt wird. Dieser Teil wird komprimiert und kondensiert als Rücklauf auf den Kopf der Trennsäule aufgegeben.

Der Erfindung lag die Aufgabe zugrunde, ein Verfahren der eingangs genannten Art so auszugestalten, daß die Abtrennung von $C_{2+}$- bzw. $C_{3+}$- oder überwiegend $C_4$-Kohlenwasserstoffen aus einem außerdem leichtere Komponenten enthaltenden Gasgemisch in hoher Ausbeute und starker Anreicherung auf möglichst einfache Weise erreicht wird.

Diese Aufgabe wird erfindungsgemäß durch das in den Ansprüchen gekennzeichnete Verfahren

gelöst.

Das zur $C_4$-Abtrennung kommende Gasgemisch sollte so beschaffen sein, daß die $C_4$-Kohlenwasserstoffe mehr als 50 mol-% der im Gasgemisch enthaltenen Kohlenwasserstoffe darstellen.

Beim erfindungsgemäßen Verfahren wird das Gasgemisch zunächst so weit unter seinen Taupunkt abgekühlt, daß ein Teil der $C_{2+}$- bzw. $C_{3+}$- oder $C_4$-Kohlenwasserstoffe als Kondensat ausfällt. Diese Kühlung wird jedoch nur so weit geführt, daß der Kondensatanfall relativ gering ist und unter 40% der insgesamt abzutrennenden Menge liegt, insbesondere unter 30% und vorzugsweise unter 20% der abzukühlenden Kohlenwasserstoffmenge. Liegt die Kondensatmenge in höheren Bereichen, wird die darin gelöste Menge an leichten Komponenten in der Regel so groß sein, daß die gewünschte Produktreinheit der $C_{2+}$- bzw. $C_{3+}$- oder $C_4$-Fraktion nicht erreicht wird, da das Kondensat nicht oder zumindest nur in geringem Umfang in der Trennsäule zerlegt wird, weil die Einspeisung schon in den unteren Säulenbereich erfolgt.

Für eine günstige Abkühlung ist es in vielen Fällen sinnvoll, den Taupunkt des Gasgemisches um höchstens 20°C, vorzugsweise um höchstens 10°C zu unterschreiten, sollen $C_4$-Kohlenwasserstoffe abgetrennt werden. Des weiteren ist sinnvoll, den Taupunkt des Gasgemisches um höchstens 80°C, vorzugsweise um höchstens 60°C zu unterschreiten, sofern die Abtrennung von $C_{3+}$-Kohlenwasserstoffen angestrebt wird und um höchstens 120°C, vorzugsweise um höchstens 100°C unter den Taupunkt abzukühlen, sofern $C_{2+}$-Kohlenwasserstoffe abgetrennt werden sollen. Dabei wird angenommen, daß der Gehalt an höheren Kohlenwasserstoffen im Vergleich zum Gehalt an $C_3$- bzw. $C_2$-Kohlenwasserstoffen relativ gering ist. Bei hohen Anteilen schwererer Kohlenwasserstoffe die ja zu einem hohen Taupunkt des Gases führen, können dagegen auch stärkere Taupunktsunterschreitungen erfolgen, wobei die Unterkühlung stets so weit geführt werden sollte, bis ein gewünschter Anteil der abzutrennenden $C_3$- bzw. $C_2$-Kohlenwasserstoffe kondensiert ist.

Die nachfolgende Rektifikation zeichnet sich beim erfindungsgemäßen Verfahren durch relativ kleine Temperaturdifferenzen zwischen dem Kopf und dem Sumpf der Rektifiziersäule aus. Diese Temperaturdifferenz liegt üblicherweise unter 25°C, vorzugsweise unter 20°C, beispielsweise bei 15°C. Zur Durchführung des Verfahrens eignet sich eine einfache Trennsäule mit mindestens zwei Gleichgewichtsstufen, beispielsweise eine Säule mit zwei bis zehn Böden, vorzugsweise mit drei bis sechs Böden bzw. mit einer Schüttung, die einer derartigen Bodenzahl entspricht.

Die vom Kopf der Trennsäule abgezogene gasförmige Fraktion enthält noch einen Teil $C_{2+}$- bzw. $C_{3+}$- oder $C_4$-Kohlenwasserstoffe, der zur Erzielung einer gewünschten hohen Ausbeute zurückgewonnen werden muß. Dazu wird die gasförmige Fraktion in einem Wärmetauscher durch indirekten Wärmetausch weiter abgekühlt und partiell kondensiert. Die Abkühlung wird so weit geführt, daß dabei alle noch rückzugewinnenden Bestandteile schwerer Kohlenwasserstoffe im Kondensat anfallen, das dann als Rücklauf auf den Kopf der Trennsäule gegeben wird. Welche zusätzliche Abkühlung durch die weitere Abkühlung erzielt wird, hängt von der gewünschten Ausbeute an schweren Kohlenwasserstoffen sowie von der im Einzelfall vorliegenden Gaszusammensetzung ab. Typische Werte für die weitere Temperaturabsenkung liegen bei mindestens 30°C, vorzugsweise bei mehr als 40°C, beispielsweise bei 50°C.

Beim erfindungsgemäßen Verfahren steht der Rücklauf für die Rektifikation bereits vollständig am obersten Boden der Rektifiziersäule zur Verfügung. Damit werden Wärme und Stoffübergang für den Trennprozeß entkoppelt, wie es dem speziellen Kondensationsverlauf des zu zerlegenden Gasgemisches entspricht.

Das bei der weiteren partiellen Kondensation abgetrennte und zur Trennsäule zurückgeführte Kondensat wird in günstiger Weiterbildung der Erfindung vor der Einspeisung in die Trennsäule wieder angewärmt, um auf diese Weise die Kälterückgewinnung des Verfahrens zu verbessern.

Der wesentliche, der Erfindung zugrundeliegende Gedanke besteht darin, daß die Ausbeute der abzutrennenden schweren Kohlenwasserstoff ($C_{2+}$, $C_3$ bzw. $C_4$) durch Wahl der Temperatur bei der weiteren Abkühlung und der Gehalt an leichten Kohlenwasserstoffen ($C_1$- bzw. $C_2$- oder $C_3$-Kohlenwasserstoffe) durch Wahl der bei der Vorkühlung einzustellenden Temperatur unabhängig voneinander eingestellt werden können. Dies ermöglicht, den Trennprozeß trotz seiner einfachen Verfahrensführung sowohl mit hoher Ausbeute als auch mit relativ hoher Produktreinheit durchzuführen. Die Konzentration der leichter als $C_2$-, $C_3$- bzw. als $C_4$-Kohlenwasserstoffe siedenden Komponenten im Kondensat, das aus dem unteren Bereich der Trennsäule abgezogen wird, kann unter 20%, vorzugsweise unter 10%, insbesondere unter 5% eingestellt werden. Andererseits kann die weitere Abkühlung der aus dem oberen Bereich der Trennsäule abgezogenen gasförmigen Fraktion so weit durchgeführt werden, daß der Gehalt an $C_{2+}$- bzw. $C_{3+}$- oder $C_4$-Kohlenwasserstoffen in der dabei nicht kondensierten Fraktion unter 15%, vorzugsweise unter 10%, insbesondere unter 5% liegt. Bei Durchführung des erfindungsgemäßen Verfahrens sind $C_{2+}$-, $C_{3+}$- bzw. $C_4$-Ausbeuten von 80%, vorzugsweise von über 90%, insbesondere von über 95% bei hoher Produktreinheit erzielbar.

In einer weiteren Ausgestaltung des erfindungsgemäßen Verfahrens kann die beim weiteren Abkühlen noch nicht kondensierte Fraktion einer weiteren dritten Kühlstufe zugeführt und dort erneut einer partiellen Kondensation unterworfen werden. Diese Verfahrensführung ist beispielsweise dann zweckmäßig, wenn das zu zerlegende Gas einen beträchtlichen Wasserstoffanteil hat und der Wasserstoff in angereicherter Form als weitere Produktfraktion gewonnen werden soll. Die weitere Abkühlung kann dann so weit durchgeführt werden, daß alle übrigen Komponenten so weit auskondensieren, daß eine gewünschte Wasserstoffreinheit erreicht wird.

Ebenso kann der bei der weiteren Abkühlung des Gasgemisches nicht kondensierte Anteil in günstiger Ausgestaltung der Erfindung im Wärmetausch gegen die abzukühlende gasförmige Fraktion und danach im Wärmetausch gegen das abzukühlende Gasgemisch angewärmt werden. Er steht danach bei annähernd Umgebungstemperatur als weitere Produktkomponente zur Verfügung.

Zur Deckung des Kältebedarfs des Verfahrens ist es zweckmäßig, die nach der letzten Kühlstufe gasförmig verbliebene Fraktion arbeitsleistend zu entspannen und danach gegen die abzukühlenden Verfahrensströme anzuwärmen. Sofern die dadurch erzielte Kühlwirkung nicht zur Deckung des Kältebedarfs des Verfahrens ausreicht, kann zusätzliche Kühlung durch Fremdkälte, beispielsweise durch einen Kühlkreislauf, bereitgestellt werden. Selbstverständlich ist es auch möglich, den gesamten Kältebedarf durch Fremdkälte zu decken, falls im Einzelfall die nicht kondensierte Fraktion einer weiteren Verarbeitung bei tiefer Temperatur zugeführt werden soll.

Weitere Einzelheiten der Erfindung werden nachfolgend anhand der in den Figuren schematisch dargestellten Ausführungsbeispiele erläutert.

Es zeigen :
Figur 1 eine erste Ausführungsform der Erfindung ;
Figur 2 eine weitere Ausführungsform der Erfindung mit einer nachgeschalteten dritten Kühlstufe und
Figur 3 eine Abwandlung der in Figur 2 dargestellten Ausführungsform der Erfindung ;
Figur 4 eine Ausführungsform der Erfindung, bei welcher $C_4$-Kohlenwasserstoffe abgetrennt werden.

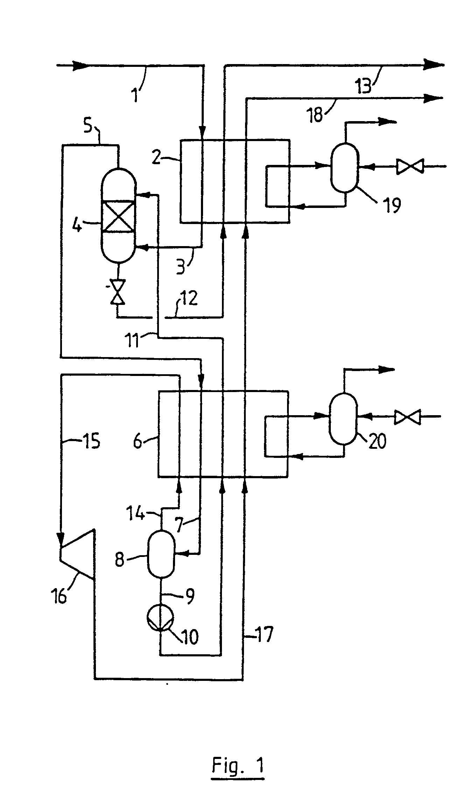

Bei dem in Figur 1 dargestellten Ausführungsbeispiel wird das zu zerlegende Gasgemisch über Leitung 1 zunächst einem Vorkühl-Wärmetauscher 2 zugeführt und darin so weit abgekühlt, daß ein Teil der abzutrennenden schweren Kohlenwasserstoffe partiell kondensiert. Das Gemisch wird anschließend über Leitung 3 in den unteren Bereich einer Trennsäule 4 geleitet. Die nicht kondensierten Anteile des Gasgemisches durchströmen eine in der Trennsäule 4 angeordnete Schüttung, wobei sie im Gegenstrom zu abwärts fließender Rücklaufflüssigkeit einen Stoffaustausch vornehmen, so daß weitere schwere Komponenten aus dem Gasgemisch abgetrennt werden. Die vom Kopf der Trennsäule 4 abgezogene gasförmige Fraktion wird über Leitung 5 einem weiteren Wärmetauscher 6 zugeleitet und darin so weit abgekühlt, daß die in der gasförmigen Fraktion noch verbliebenen schweren Kohlenwasserstoffe so weit auskondensieren, wie es der gewünschten Ausbeute entspricht. Der partiell kondensierte Strom gelangt über Leitung 7 in einen Abscheider 8, in dem das gebildete Kondensat abgetrennt, über Leitung 9 abgezogen und mittels der Pumpe 10 durch den Wärmetauscher 6 gefördert wird, bevor es schließlich über Leitung 11 auf den Kopf der Trennsäule 4 als Rücklaufflüssigkeit aufgegeben wird. Die im Kondensat enthaltenen leichten Bestandteile werden beim Durchströmen der Trennsäule 4 wieder abgetrennt und verlassen die Säule über Leitung 5, während die darin enthaltenen schweren Komponenten in den Sumpfbereich gelangen und von dort, gemeinsam mit den bei der Vorkühlung 2 kondensierten Bestandteilen, als Kopfproduktstrom über Leitung 12 abgezogen wrden. Der Produktstrom wird nach Anwärmung im Wärmetauscher 2 schließlich über Leitung 13 aus der Anlage abgeführt.

Die im Abscheider 8 gasförmig verbliebenen Anteile werden über Leitung 14 abgezogen, im Wärmetauscher 6 angewärmt, danach über Leitung 15 einer Entspannungsturbine 16 zugeführt und darin kälteleistend entspannt. Das entspannte Gas gelangt über Leitung 17 zunächst zum Wärmetauscher 6 und gibt nach Durchströmen desselben schließlich seine Restkälte im Wärmetauscher 2 ab, bevor es über Leitung 18 als Restgas aus der Anlage abgezogen wird. Sofern die in den anzuwärmenden Verfahrensströmen enthaltene Kälte nicht zur Abkühlung des zu zerlegenden Gasgemisches ausreicht, können zusätzliche Kältekreisläufe 19 bzw. 20 vorgesehen sein.

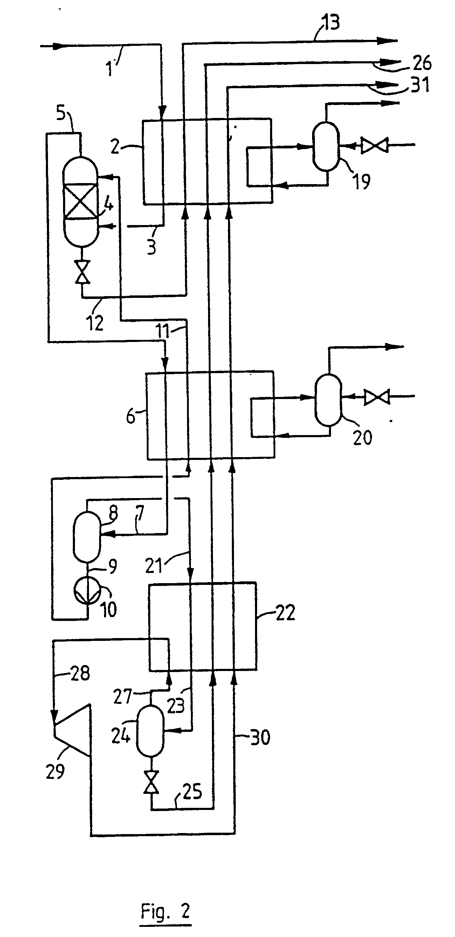

Bei der in Figur 2 dargestellten Ausführungsform der Erfindung wird die vom Kopf des Abscheiders 8 abgezogene, bei der weiteren partiellen Kondensation nicht kondensierte gasförmige Fraktion noch einem weiteren Kondensationsschritt unterzogen. Hierzu wird die gasförmige Fraktion über Leitung 21 abgezogen in einem weiteren Wärmetauscher 22 so weit abgekült, daß die verbleibende gasförmige Fraktion im wesentlichen nur noch eine bzw. mehrere gewünschte Komponenten enthält, die dann als weitere Produktfraktion separat abgezogen werden kann. Das partiell kondensierte Gas gelangt über Leitung 23 in einen weiteren Abscheider 24, aus dessen unterem Bereich über Leitung 25 das Kondensat abgezogen und nach Erwärmung in den Wärmetauschern 22, 6 und 2 über Leitung 26 als Restgas abgeführt wird. Die im Abscheider 24 anfallende gasförmige Fraktion wird über Leitung 27 dem Wärmetauscher 22 zugeführt, darin teilweise wieder erwärmt, anschließend über Leitung 28 einer Expansionsturbine 29 zugeführt und arbeitsleistend entspannt. Die dabei gewonnene Kälte wird durch Wärmetausch gegen abzukühlende Verfahrensströme zurückgewonnen, wozu das Gasgemisch über Leitung 30 den Wärmetauschern 22, 6 und 2 zugeführt wird, bevor es schließlich über Leitung 31 als Produktstrom abgezogen wird.

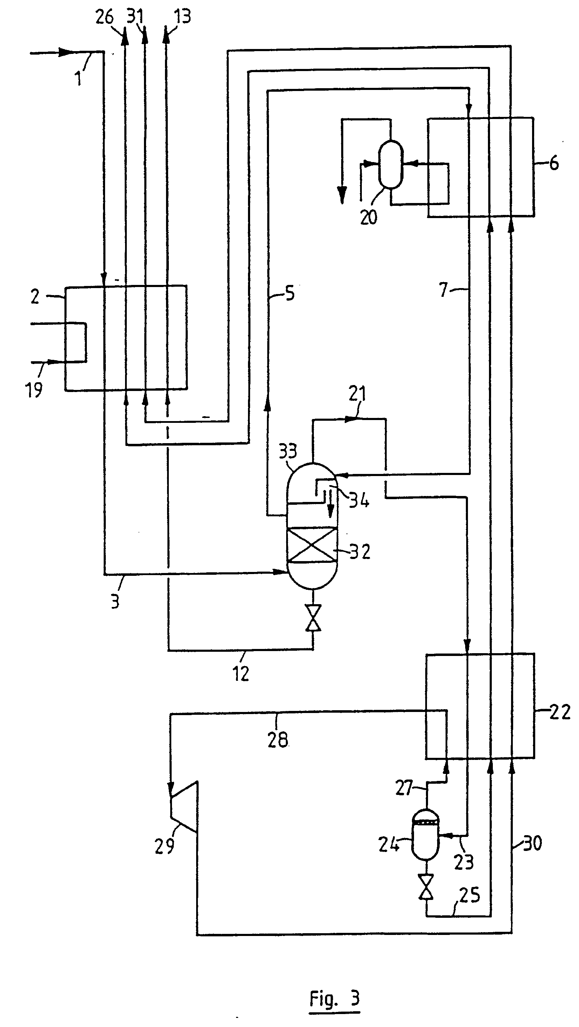

Das in der Figur 3 dargestellte Ausführungsbeispiel unterscheidet sich von demjenigen gemäß Figur 2 im. wesentlichen dadurch, daß die Trennsäule 4 und der Abscheider 8 zu einer konstruktiven Einheit zusammengefaßt worden sind und der Wärmetausch des im Abscheider 8 gemäß Figur 2 anfallenden Kondensats mit der abzukühlenden gasförmigen Fraktion aus Leitung 5 entfällt. Dadurch wird zwar die Kälterückgewinnung des Verfahrens etwas verschlechtert, doch der konstruktive Aufwand dafür verringert, da der separate Abscheider 8, die Förderpumpe 10 sowie ein Strömungsquerschnitt im Wärmetausche 6, der üblicherweise ein Plattenwärmetauscher sein wird, entfallen. Beim Verfahren gemäß Figur 3 tritt das Gasgemisch nach der Abkühlung im Wärmetauscher 2 über Leitung 3 in den unteren Bereich einer Trennsäule 32, die ebenso wie die Trennsäule 4 gemäß Figuren 1 und 2 betrieben wird. In die Trennsäule 32 ist jedoch am Kopf der Abscheider 8 gemäß Figuren 1 und 2 integriert. Die über einen Seitenanstich abgezogene Kopffraktion gelangt über Leitung 5 wiederum zum Wärmetauscher 6, wird darin partiell kondensiert und gelangt über Leitung 7 in den am Kopf der Trennsäule befindlichen Abscheider 33. Das im Wärmetauscher 6 gebildete Kondensat staut sich im Abscheider 33 an einem Überlaufwehr und gelangt von dort, wie durch den Pfeil 34 angedeutet, direkt auf den Kopf der Trennsäule 32. Die bei der partiellen Kondensation im Wärmetauscher 6 gasförmig verbliebene Fraktion tritt über Leitung 21 aus der Trennsäule 32 aus und wird genauso weiterverarbeitet, wie bereits mit Bezug auf Figur 2 beschrieben.

In einem konkreten Ausführungsbeispiel der Erfindung wird bei einer Verfahrensführung gemäß Figur 3 ein Gasgemisch, das 80,8 mol-% Wasserstoff, 7,1 mol-% Methan, 5,6 mol-% Ethan, 4,0 mol-% Propan, 1,6 mol-% Butan und 0,9 mol-% $C_{5+}$-Kohlenwasserstoffe enthält, bei einer Temperatur von 516 K und einem Druck von 38 bar über Leitung 1 herangeführt. Im Wärmetauscher 2 wird das Gasgemisch auf 256 K abgekühlt, wobei etwa 3,3% des Gasgemisches partiell kondensieren. Vom Kopf der Trennsäule 32 wird über Leitung 5 eine gasförmige Fraktion bei einer Temperatur von 248 K abgezogen, die 84,7 mol-% Wasserstoff, 7,3 mol-% Methan, 5,3 mol-% Ethan, 2,6 mol-% Propan und 0,1 mol-% Butan enthält. Diese Fraktion wird im Wärmetauscher 6 auf 232 K abgekühlt, wobei etwa 2,8% der Fraktion kondensiert, und danach über Leitung 7 in den Abscheider 33 am Kopf der Trennsäule 32 geführt wird. Die auf den Säulenkopf gegebene Flüssigkeit enthält 2,2 mol-% Wasserstoff, 2,5 mol-% Methan, 22,0 mol-% Ethan, 61,4 mol-% Propan, 11,8 mol-% Butan und 0,1 mol-% $C_{5+}$-Kohlenwasserstoffe. Vom Sumpf der Trennsäule 32 wird über Leitung 12 ein Produktstrom bei einer Temperatur von 255 K abgezogen, der 2,1 mol-% Wasserstoff, 1,8 mol-% Methan, 12,5 mol-% Ethan, 32,8 mol-% Propan, 32,7 mol-% Butan und 18,1 mol-% $C_{5+}$-Kohlenwasserstoffe enthält. Über Leitung 21 wird eine gasförmige Fraktion aus dem Abscheider 33 abgezogen, die 84,7 mol-% Wasserstoff, 7,33 mol-% Methan, 5,3 mol-% Ethan, 2,6 mol-% Propan und 0,1 mol-% Butan enthält. Nach weiterer Abkühlung im Wärmetauscher 22 verbleibt eine wasserstoffreiche gasförmige Fraktion, die in der Entspannungsturbine 29 auf 20,5 bar entspannt wird und neben 96,2 mol-% Wassertoff nur noch 3,8 mol-% Ethan enthält. Das im Abscheider 24 abgetrennte Kondensat enthält 10,6 mol-% Wasserstoff, 30,2 mol-% Methan, 39,4 mol-% Ethan, 19,1 mol-% Propan und 0,7 mol-% Butan. Diese Fraktion wird nach ihrer Anwärmung als Restgas über Leitung 26 abgezogen.

Bei diesem Ausführungsbeispiel wird von einem bisher nur als Heizgas verwendeten Restgas ohne großen Aufwand eine $C_3$-Fraktion sowie eine Rohwasserstoff-Fraktion abgetrennt, wobei die Ausbeute an $C_{3+}$-Kohlenwasserstoffen bei 61% und die Wasserstoffausbeute bei 98,3% lag. Die für diesen konkreten Fall ausreichende, relativ niedrige $C_{3+}$-Ausbeute ist durch die relativ geringe Abkühlung der gasförmigen Fraktion aus Leitung 5 im Wärmetauscher 6 bedingt. Dies hatte im konkreten Ausführungsbeispiel zur Folge, daß über Leitung 21 noch etwa 60% des im Gasgemisch enthaltenen Propans gasförmig abgezogen wurde. Bei stärkerer Kühlung im Wärmetauscher 6 läßt sich die $C_{3+}$-Ausbeute des Verfahrens noch erheblich steigern, beispielsweise auf Werte zwischen 80 und 95%.

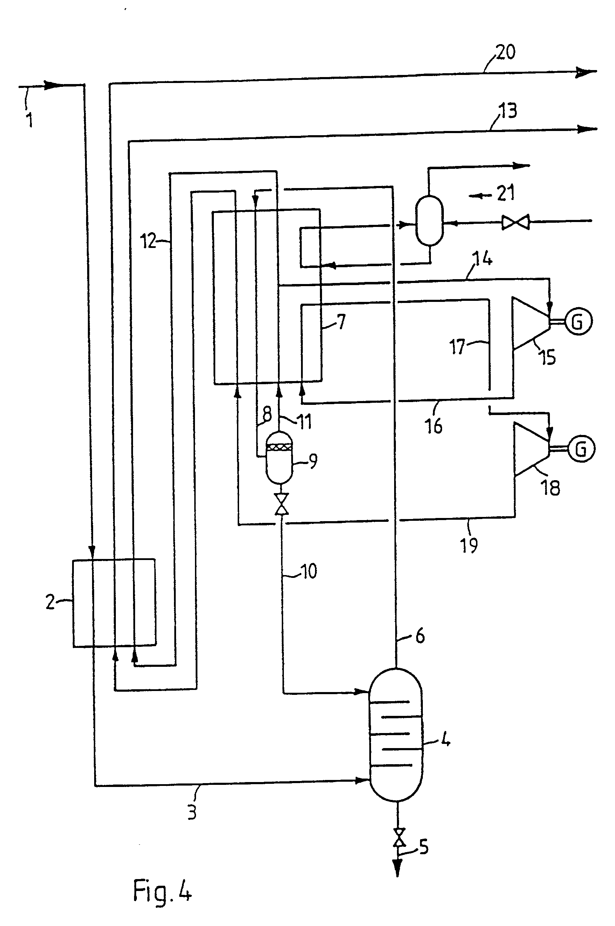

Bei dem in Figur 4 dargestellten Verfahren wird ein aus einer Isobutan-Dehydrierung kommender Einsatzstrom, der 70,5 mol-% Wasserstoff, 3,5 mol-% Methan, 0,5 mol-% $C_2$-Kohlenwasserstoffe, 2,5 mol-% $C_3$-Kohlenwasserstoffe, 9,7 mol-% $C_4$-Kohlenwasserstoffe und 0,8 mol-% Kohlendioxid, 0,7 mol-% Kohlenmonoxid und 11,8 mol-% Stickstoff enthält, über Leitung 1 zugeführt und in einem Wärmetauscher 2 von einer Temperatur von 290 K auf 283 K abgekühlt. Das über Leitung 1 herangeführte Gasgemisch hat einen Druck von 29,6 bar und befindet sich am Taupunkt. Durch die Vorkühlung im Wärmetauscher 2 kondensieren etwa 2,3% des Gasstroms. Der Gasstrom wird über Leitung 3 in den unteren Bereich einer Rektifiziersäule 4 eingespeist, in der die Abtrennung der $C_4$-Kohlenwasserstoffe von den übrigen Komponenten erfolgt. Die abzutrennenden $C_4$-Kohlenwasserstoffe sammeln sich im Sumpf der Rektifiziersäule und werden über Leitung 5 als Produktstrom abgezogen. Dieses Produkt enthält neben 85,4 mol-% $C_4$-Kohlenwasserstoffen noch 1,8 mol-% Wasserstoff, 0,6 mol-% Methan, 0,4 mol-% $C_2$-Kohlenwasserstoffe, 10,4 mol-% $C_2$-Kohlenwasserstöffe, 0,4 mol-% Kohlendioxid und 0,6 mol-% Stickstoff. Der über Leitung 5 bei einer Temperatur von 282,5 K abgezogene Produktstrom enthält 99% der im zugeführten Gasgemisch enthaltenen $C_4$-Kohlenwasserstoffe.

Am Kopf der Rektifiziersäule 4 wird über Leitung 6 eine gasförmige Fraktion bei einer Temperatur von 265 K abgezogen, die 79,1 mol-% Wasserstoff, 3,9 mol-% Methan, 0,5 mol-% $C_2$-Kohlenwasserstoffe, 1,5 mol-% $C_3$-Kohlenwasserstoffe, 0,1 mol-% $C_4$-Kohlenwasserstoffe, 0,9 mol-% Kohlendioxid, 0,8 mol-% Kohlenmonoxid und 13,2 mol-% Stickstoff enthält. Dieses Gas wird in einem Wärmetauscher 7 auf eine Temperatur von 211 K abgekühlt, wobei etwa 10,2% des Gasgemisches kondensieren. Nachdem das Gemisch über Leitung 7 in einen Abscheider 9 eingetreten ist, findet hier eine Phasentrennung statt. Der kondensierte Anteil wird über Leitung 10 als Rücklauf auf den Kopf der Rektifiziersäule 4 zurückgegeben, während der nicht kondensierte Anteil über Leitung 11 aus dem oberen Bereich des Abscheiders 9 abgezogen wird. Dieses Gas enthält 71,2 mol-% Wasserstoff, 3,6 mol-% Methan, 0,7 mol-% $C_2$-Kohlenwasserstoffe, 8,4 mol-% $C_3$-Kohlenwasserstoffe, 2,4 mol-% $C_4$-Kohlenwasserstoffe, 1,0 mol-% Kohlendioxid, 0,7 mol-% Kohlenmonoxid und 11,9 mol-% Stickstoff. Es wird im Wärmetauscher 7 gegen die abzukühlende gasförmige Fraktion aus der Rektifiziersäule 4 angewärmt und gelangt danach über Leitung 12 zum Wärmetauscher 2, in dem sie weiter gegen das abzukühlende Gasgemisch angewärmt wird, bevor sie schließlich über Leitung 13 als Restgas-Fraktion bei einer Temperatur von 287 K und unter einem Druck von 28,5 bar abgegeben wird. Zur Deckung des Kältebedarfs des Verfahrens wird über Leitung 14 ein Teilstrom des im Wärmetauscher 7 teilweise angewärmten, über Leitung 11 abgezogenen Restgasstroms abgezweigt und einer arbeitsleistenden Entspannung unterworfen. Dazu wird das Gas in einer Entspannungsftrbine 15 zunächst auf einen Zwischendruck entspannt, dann über Leitung 16 erneut durch den Wärmetauscher 7 geführt und nach Teilerwärmung in diesem über Leitung 17 einer zweiten Entspannungsturbine 18 zugeführt, in der es weiter auf einen tieferen Druck entspannt und danach über Leitung 19 abgezogen wird. Das kalte Gas in Leitung 19 wird zunächst im Wärmetauscher 7 und danach im Wärmetauscher 2 gegen abzukühlende Verfahrensströme angewärmt, bevor es schließlich über Leitung 20 als Niederdruckrestgas abgegeben wird.

Zur Deckung eines weiteren Kältebedarfs im Wärmetauscher 7 kann ein durch 21 angedeuteter Kältekreislauf vorgesehen sein.

**Ansprüche**

1. Verfahren zum Abtrennen höherer Kohlenwasserstoffe aus einem diese und leichter siedende Komponenten enthaltenden Gasgemisch durch rektifikatorische Zerlegung, indem das Gasgemisch partiell kondensiert und einer Trennsäule zugeleitet wird, an deren Sumpf eine an höheren Kohlenwasserstoffen reiche Fraktion und an deren Kopf eine an leichter siedenden Komponenten reiche Fraktion abgezogen werden, wobei ein Teil der Kopffraktion kondensiert und als Rücklauf auf den Kopf der Trennsäule gegeben wird, dadurch gekennzeichnet, daß das Gasgemisch (1) zunächst so weit in einem Wärmeaustausche (2) abgekühlt wird, daß ein kleinerer Teil der $C_{2+}$-, $C_{3+}$- bzw. der $C_4$-Kohlenwasserstoffe, von unter 40% der abzutrennenden Menge, kondensiert (3), wonach es in den unteren Bereich einer Trennsäule (4/32) eingespeist wird, daß aus dem oberen Bereich der Trennsäule eine gasförmige $C_{2+}$- bzw. $C_{3+}$- oder $C_4$-Kohlenwasserstoffe enthaltende Fraktion (5) abgezogen wird, die in einem weiteren Wärmetauscher (6) weiter abgekühlt und partiell kondensiert (7) wird, wonach der partiell kondensierte Anteil (9/34), die noch rückzugewinnenden Bestandteile schwerer Kohlenwasserstoffe enthaltend, als Rücklauf auf den Kopf der Trennsäule (4/32) gegeben wird und aus dem Sumpf der Trennsäule $C_{2+}$-, $C_{3+}$- bzw. $C_4$-Kohlenwasserstoffe als Produktstrom (12) abgezogen werden.

2. Verfahren nach Anspruch 1, dadurch gekennzeichnet, daß der partiell kondensierte Anteil (9) der gasförmigen Fraktion (5) vor Einspeisung in die Trennsäule angewärmt wird (6).

3. Verfahren nach Anspruch 1 oder 2, dadurch gekennzeichnet, daß die Trennsäule mit einer Temperaturdifferenz von weniger als 25°C, vorzugsweise von weniger als 20°C, zwischen dem oberen und dem unteren Bereich betrieben wird.

4. Verfahren nach einem der Ansprüche 1 bis 3, dadurch gekennzeichnet, daß durch die weitere Abkühlung (6) der gasförmigen Fraktion (5) vom Kopf der Trennsäule (4) eine Temperaturabsenkung von mindestens 30°C, vorzugsweise von mehr als 40°C, erzielt wird.

5. Verfahren nach einem der Ansprüche 1 bis 4, dadurch gekennzeichnet, daß das Gasgemisch (1) zunächst nur soweit abgekühlt wird, daß im dabei gebildeten Kondensat (3) die Konzentration der leichter als $C_2$-, $C_3$- bzw. $C_4$-Kohlenwasserstoffe siedenden Komponenten unterhalb der für die $C_{2+}$-, $C_{3+}$- bzw. $C_4$-Kohlenwasserstoffe-Produktfraktion (12) zulässigen Werte liegt.

6. Verfahren nach einem der Ansprüche 1 bis 5, dadurch gekennzeichnet, daß die Konzentration der leichter als $C_2$-, $C_3$- bzw. $C_4$-Kohlenwasserstoffe siedenden Komponenten im Kondensat (3) unter 20%, vorzugsweise unter 10%, insbesondere unter 5%, liegt.

7. Verfahren nach einem der Ansprüche 1 bis 6, dadurch gekennzeichnet, daß die weitere Abkühlung (6) der aus dem oberen Bereich der Trennsäule abgezogenen gasförmigen Fraktion (5) so weit erfolgt, daß die Menge an $C_{2+}$-, $C_{3+}$- bzw. $C_4$-Kohlenwasserstoffen in der dabei nichtkondensierenden Fraktion nicht größer ist als die für die Durchführung des Verfahrens tolerierten Ausbeuteverluste.

8. Verfahren nach einem der Ansprüche 1 bis 7, dadurch gekennzeichnet, daß die weitere Abkühlung (6) der aus dem oberen Bereich der Trennsäule abgezogenen gasförmigen Fraktion (5) so weit erfolgt, daß der Gehalt an $C_{2+}$- bzw. $C_{3+}$- oder $C_4$-Kohlenhwasserstoffen in der dabei nicht kondensierten Fraktion unter 15%, vorzugsweise unter 10%, insbesondere unter 5%, liegt.

9. Verfahren nach einem der Ansprüche 1 bis 8, dadurch gekennzeichnet, daß die beim weiteren Abkühlen (6) nicht kondensierte Fraktion (21) einer dritten Kühlstufe (22) zugeführt und einer weiteren partiellen Kondensation unterworfen wird.

10. Verfahren nach einem der Ansprüche 1 bis 9, dadurch gekennzeichnet, daß die nach der letzten Kühlstufe gasförmig verbliebene Fraktion (14/27) arbeitsleistend entspannt (16/29) und danach gegen abzukühlende Verfahrensströme angewärmt wird (6, 2/22, 6, 2).

11. Verfahren nach einem der Ansprüche 1 bis 10, dadurch gekennzeichnet, daß der bei der weiteren Abkühlung der gasförmigen Fraktion (5) vom Kopf der Trennsäule (4/32) nicht kondensierte Teil (15, 17/28, 30) im Wärmetausch gegen die abzukühlende gasförmige Fraktion (5) und danach im Wärmetausch gegen das abzukühlende Gasgemisch (1) angewärmt wird.

12. Verfahren nach einem der Ansprüche 1 bis 11, dadurch gekennzeichnet, daß die im Gasgemisch (1) enthaltenen Kohlenwasserstoffe zu 50 bis 70% aus $C_4$-Kohlenwasserstoffen, zu 15 bis 40% aus Methan und zu 0 bis 25% aus $C_2$- und $C_3$-Kohlenwasserstoffen bestehen.

## Revendications

1. Procédé de séparation d'hydrocarbures supérieurs d'un mélange gazeux contenant ces hydrocarbures et des composants à plus bas point d'ébullition par séparation et rectification, dans lequel on condense partiellement le mélange gazeux et on le fait passer dans une colonne de séparation en cuve de laquelle on prélève une fraction riche en hydrocarbures supérieurs et en tête de laquelle on prélève une fraction riche en composants de plus bas point d'ébullition, une partie de la fraction de tête étant condensée et faisant office de reflux en tête de la colonne de séparation, caractérisé en ce que le mélange gazeux (1) est tout d'abord refroidi dans un échangeur de chaleur (2) jusqu'au point où une petite proportion des hydrocarbures en $C_2^+$, $C_3^+$ ou $C_4$ respectivement, inférieure à 40% du mélange à séparer est condensée (3) puis introduit dans la partie inférieure d'une colonne de séparation (4/32), en ce que l'on retire en tête de la colonne de séparation une fraction (5) gazeuse contenant des hydrocarbures en $C_2^+$, $C_3^+$ ou $C_4$ que l'on soumet à un autre refroidissement dans un échangeur de chaleur supplémentaire (6) et condense (7) partiellement, la partie (9/34) partiellement condensée qui contient encore des constituants hydrocarbonés lourds à récupérer étant retournée comme reflux en tête de la colonne de distillation (4/32) et en ce que l'on retire en cuve de la colonne de distillation les hydrocarbures en $C_2^+$, $C_3^+$ et $C_4$ formant le courant (12) produit.

2. Procédé selon la revendication 1, caractérisé en ce que la fraction partiellement condensée (9) de la fraction gazeuse (5) est réchauffée (en 6) avant introduction dans la colonne de séparation.

3. Procédé selon la revendication 1 ou 2, caractérisé en ce que la colonne de séparation opère avec une différence de température entre la cuve et la tête, inférieure à 25°C, et de préférence inférieure à 20°C.

4. Procédé selon l'une quelconque des revendications 1 à 3, caractérisé en ce que, par le refroidissement supplémentaire (6) de la fraction gazeuse (5) de la tête de la colonne de séparation (4), on obtient un abaissement de la température d'au moins 30°C, de préférence supérieur à 40°C.

5. Procédé selon l'une quelconque des revendications 1 à 4, caractérisé en ce que le refroidissement du mélange gazeux (1) est suffisamment poussé pour que, dans le condensat (3) ainsi formé, la concentration des composants à points d'ébullition plus faibles que ceux des hydrocarbures en $C_2$, $C_3$ et/ou $C_4$, soit inférieure à la valeur admissible pour la fraction de produit (12) des hydrocarbures en $C_2^+$, $C_3^+$ et/ou $C_4$.

6. Procédé selon l'une quelconque des revendications 1 à 5, caractérisé en ce que la concentration en composants à points d'ébullition inférieurs à ceux des hydrocarbures en $C_2$, $C_3$ et/ou $C_4$ dans le condensat (3) est inférieure à 20%, de préférence inférieure à 10%, notamment inférieure à 5%.

7. Procédé selon l'une quelconque des revendications 1 à 6, caractérisé en ce que le refroidissement supplémentaire (6) de la fraction gazeuse (5) prélevée dans la partie supérieure de la colonne de séparation est suffisamment poussé pour que la quantité d'hydrocarbures en $C_2^+$, $C_3^+$ et/ou $C_4$ dans la fraction qui n'est alors pas condensée ne soit pas supérieure aux pertes de rendement tolérés pour la mise en oeuvre du procédé.

8. Procédé selon l'une quelconque des revendications 1 à 7, caractérisé en ce que la poursuite du refroidissement (6) de la fraction gazeuse (5) prélevée en tête de la colonne de séparation est poussée de façon à ce que la teneur en hydrocarbures en $C_2^+$

et/ou $C_3^+$ ou $C_4$ dans la fraction qui n'est alors pas condensée soit inférieure à 15%, de préférence inférieure à 10%, notamment inférieure à 5%.

9. Procédé selon l'une quelconque des revendications 1 à 8, caractérisé en ce que la fraction (21) non condensée lors de la poursuite du refroidissement (6) est amenée à une troisième étape de refroidissement (22) et elle est soumise à une condensation partielle supplémentaire.

10. Procédé selon l'une quelconque des revendications 1 à 9, caractérisé en ce que la fraction gazeuse subsistant (14/27) après la dernière étape de refroidissement est détendue (16/29) avec production de travail et elle est réchauffée en échange de chaleur avec les courants de produit à refroidir (6, 2/22, 6, 2).

11. Procédé selon l'une quelconque des revendications 1 à 10, caractérisé en ce que la fraction non condensée (15, 17/28, 30) sortant de la tête de la colonne de séparation (4/32) lors du refroidissement supplémentaire de la fraction gazeuse (5) est réchauffée en échange de chaleur avec la fraction gazeuse à refroidir (5) et ensuite en échange de chaleur avec le mélange gazeux (1) à refroidir.

12. Procédé selon l'une quelconque des revendications 1 à 11, caractérisé en ce que les hydrocarbures contenus dans le mélange gazeux (1) sont constitués à concurrence de 50 à 70% d'hydrocarbures en $C_4$, de 15 à 40% de méthane et de 0 à 25% d'hydrocarbures en $C_2$ et $C_3$.

## Claims

1. A process for separating heavier hydrocarbons from a gas mixture which contains the latter and lower-boiling components, by rectificational separation, in that the gas mixture is partially condensed and fed to a separating column, at the sump of which a fraction rich in heavier hydrocarbons is discharged and at the head of which a fraction rich in lower-boiling components is discharged, wherein a part of the head fraction is condensed and fed as reflux to the head of the separating column, characterised in that the gas mixture (1) is firstly cooled in a heat exchanger (2) until a smaller part of the $C_{2+}$, $C_{3+}$ or $C_4$-hydrocarbons, of less than 40% of the quantity which is to be separated, condenses (3), whereupon it is input into the lower region of a separating column (4/32), that a gaseous fraction (5) containing $C_{2+}$, $C_{3+}$ or $C_4$-hydrocarbons is discharged from the upper region of the separating column, which fraction (5) is further cooled in a further heat exchanger (6) and partially condensed (7), whereupon the partially condensed constituent (9/34) containing constituents, still to be regained, of heavy hydrocarbons, is fed as reflux to the head of the separating column (4/32) and $C_{2+}$, $C_{3+}$ or $C_4$-hydrocarbons are discharged from the sump of the separating column as a product stream (12).

2. A process as claimed in claim 1, characterised in that the partially condensed constituent (9) of the gaseous fraction (5) is heated (6) before it is fed into the separating column.

3. A process as claimed in claim 1 or claim 2, characterised in that the separating column is operated with a temperature difference of less than 25°C, preferably less than 20°C, between the upper and the lower regions.

4. A process as claimed in one of the claims 1 to 3, characterised in that as a result of the further cooling (6) of the gaseous fraction (5) from the head of the separating column (4) a temperature reduction of at least 30°C, preferably more than 40°C, is obtained.

5. A process as claimed in one of claims 1 to 4, characterised in that the gas mixture (1) is firstly cooled only until, in the condensate (3) which is thereby formed, the concentration of the components which are lower boiling than $C_2$, $C_3$ or $C_4$-hydrocarbons is below the values permissible for the $C_{2+}$, $C_{3+}$ or $C_4$-hydrocarbon-product fraction (12).

6. A process as claimed in one of claims 1 to 5, characterised in that the concentration in the condensate (3) of the components which are lower boiling than $C_2$, $C_3$ or $C_4$-hydrocarbons is below 20%, preferably below 10%, in particular below 5%.

7. A process as claimed in one of claims 1 to 6, characterised in that the further cooling (6) of the gaseous fraction (5) discharged from the upper region of the separating column continues until the quantity of $C_{2+}$, $C_{3+}$ or $C_4$-hydrocarbons in the fraction which does not thereby condense in no greater than the yield losses tolerated for the execution of the process.

8. A process as claimed in one of claims 1 to 7, characterised in that the further cooling (6) of the gaseous fraction (5) discharged from the upper region of the separating column continues until the content of $C_{2+}$, $C_{3+}$ or $C_4$-hydrocarbons in the fraction which does not thereby condense is less than 15%, preferably less than 10%, and in particular less than 5%.

9. A process as claimed in one of claims 1 to 8, characterised in that the fraction (21) which has not condensed during the further cooling (6) is fed to a third cooling stage (22) and subjected to additional, partial condensation.

10. A process as claimed in one of claims 1 to 9, characterised in that the fraction (14/27) which remains in gaseous form following the last cooling stage is expanded with the production of work (16/29) and is then heated (6, 2/22, 6, 2) in the presence of process streams which are to be cooled.

11. A process as claimed in one of claims 1 to 10, characterised in that the constituent (15, 17/28, 30) which does not condense in the further cooling of the gaseous fraction (5) from the head of the separating column (4/32) is heated in heat exchange with the gaseous fraction (5) which is to be cooled and then in heat exchange with the gas mixture (1) which is to be cooled.

12. A process as claimed in one of claims 1 to 11, characterised in that the hydrocarbons contained in the gas mixture (1) comprise 50% to 70% $C_4$-hydrocarbons, 15% to 40% methane and 0% to 25% $C_2$ and $C_3$-hydrocarbons.

Fig. 1

Fig. 2

Fig. 3

Fig. 4